(19) Europäisches Patentamt
European Patent Office
Office européen des brevets

(11) EP 1 436 373 B1

(12) # EUROPEAN PATENT SPECIFICATION

(45) Date of publication and mention of the grant of the patent:
**07.06.2006 Bulletin 2006/23**

(21) Application number: **02776237.6**

(22) Date of filing: **18.10.2002**

(51) Int Cl.:
*C11D 3/50* (2006.01) *A61L 9/01* (2006.01)
*C11D 3/30* (2006.01) *C11D 3/33* (2006.01)
*C11D 3/37* (2006.01)

(86) International application number:
**PCT/US2002/033377**

(87) International publication number:
**WO 2003/033635 (24.04.2003 Gazette 2003/17)**

(54) **CONTROLLED BENEFIT AGENT DELIVERY SYSTEM**
KONTROLLIERTES WIRKSTOFFABGABESYSTEM
SYSTEME D'ADMINISTRATION CONTROLE D'UN AGENT AVANTAGEUX

(84) Designated Contracting States:
**AT BE BG CH CY CZ DE DK EE ES FI FR GB GR IE IT LI LU MC NL PT SE SK TR**

(30) Priority: **19.10.2001 US 345469 P**

(43) Date of publication of application:
**14.07.2004 Bulletin 2004/29**

(73) Proprietor: **THE PROCTER & GAMBLE COMPANY**
**Cincinnati, Ohio 45202 (US)**

(72) Inventors:
- **DYKSTRA, Robert, Richard**
**Cleves, OH 45002 (US)**
- **GRAY, Lon, Montgomery**
**Florence, KY 41042 (US)**
- **MIRACLE, Gregory, Scot**
**Hamilton, OH 45013 (US)**
- **GALLON, Lois, Sara**
**Cincinnati, OH 45224 (US)**
- **MALTON, Peter, James**
**Staines, Middlesex TW18 1EW (GB)**

(74) Representative: **Howard, Phillip Jan et al**
**Procter & Gamble Technical Centres Ltd.**
**Whitley Road**
**Longbenton**
**Newcastle upon Tyne NE12 9TS (GB)**

(56) References cited:
**EP-A- 0 864 642** **EP-A- 1 067 173**
**EP-A- 1 116 788** **WO-A-01/93823**
**US-A- 4 985 402** **US-A- 4 990 494**
**US-A- 6 103 678** **US-A- 6 153 567**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 436 373 B1

## Description

### Field of the Invention

The present invention relates to benefit agent delivery systems that, when directly applied to a substrate, provide a longer benefit term than the benefit agent alone.

### Background of the Invention

It is frequently desirable or advantageous to treat the surfaces of a variety of substrates, for example fabrics, with benefit agents such as perfumes, flavors, pharmaceuticals and/or biocontrol agents including biocides, insecticides, mildewcides, and the like. Generally, the objective of such treatment is to leave deposited on the surfaces of the substrates enough benefit agent so that there is a residual benefit imparted to the substrate surface.

Products, systems and methods for depositing benefit agents onto the surfaces of substrates are known in the art. For example, in the context of fabric treatment, such as fabric laundering, a variety of products can be used to form benefit imparting aqueous washing liquors or rinse baths.

Other products that provide improved deposition onto substrate surfaces are benefit agents such as perfumes. Such products are described in PCT Patent Application Nos. WO 00/02991; WO 00/02981; WO 00/02987 and WO 00/02982. These patent publications disclose compositions wherein a residual benefit agent is realized by incorporating a reaction product formed from amine-based compounds and certain types of benefit agents that will react with such amine-based compounds into substrate treatment products.

However, notwithstanding the advances in the art, there remains a continuing need for benefit agent delivery systems that are especially effective for directly delivering residual and long-lasting benefit agents to substrates.

### Summary of the Invention

The present invention relates to a benefit agent delivery system suitable for delivering a benefit agent to a substrate, wherein the benefit agent delivery system comprises a benefit agent in the form of an aldehyde or ketone and an amine comprising an amine moiety selected from the group consisting of primary amines, secondary amines and mixtures thereof, such that when said amine and said benefit agent are directly applied to a substrate, the benefit agent provides a benefit to the substrate for a longer period of time than when said amine is not present, wherein the amine comprises, based on the total number of amine moieties in the amine, from 15% to 100% primary amine moieties, and wherein the amine comprises a hydroxy moiety.

The present invention also relates to products comprising the aforementioned delivery system and methods of using same.

### Detailed Description

Applicants' benefit delivery systems provide formulation flexibility as initial and ongoing benefit release levels can be adjusted to achieve almost any type of release profile, including profiles that are consistent with time. For example, in one aspect of Applicants' invention the amount of benefit agent that is initially released is perceptibly less than that normally released, however, such release rate is more consistent with time. In the event that a combination of high initial and sustained release is desired, the skilled artisan need only alter the level or mix of the one or more of benefit agents employed. In general, the benefit agent and amine component of the present invention should be in intimate contact for a sufficient period of time, before being applied, to provide the optimum benefit. While such contact period may vary from application to application, Applicants' have discovered that a seven day contact period is sufficient for most applications.

The components of the benefit agent delivery systems herein are selected and processed such that the resulting delivery systems are especially effective for delivering the benefit agent to a substrate that has been directly contacted with delivery system. Under such conditions, the benefit agent delivered to the substrate surface will provide its benefit thereto for a longer period of time than if no amine-based compound were present in the delivery system.

The benefit agent delivery systems of the present invention are particularly useful in various applications and/or products that are intended to be applied without dilution, such as fine fragrance perfume applications and/or products, home care perfume products, such as freshening compositions, that may comprise cyclodextrins, and that are typically applied to upholstery, carpets and other fabric articles, hard surface treating compositions, beauty care applications, such as creams, lotions, deodorants, antiperspirants, and other topical compositions, hair care compositions, such as hair spray, leave-in conditioners, and the like.

Definitions and Test Methods:

"Directly applied" and/or "delivering directly" as used herein means that a benefit agent is applied to a substrate via the benefit agent delivery system such that the benefit provided by the benefit agent is realized and/or recognized prior to dilution. For example, a benefit agent is sprayed onto a substrate and/or wiped on to a substrate, rather than having the benefit agent contact or deposit indirectly onto a substrate from a dilute solution (i.e., wash liquor).

The term "unit which can substitute for hydrogen" means "chemical moieties which can replace a hydrogen atom on a hydrocarbon chain, an aryl ring, and the like, or replace a hydrogen atom, two hydrogen atoms, or three hydrogen atoms from a carbon atom to form a moiety, or replace hydrogen atoms from adjacent carbon atoms to form a moiety." For example, a substituted unit that requires a single hydrogen atom replacement includes halogen, hydroxyl, and the like. A two hydrogen atom replacement includes carbonyl, oximino, and the like. Three hydrogen replacement includes cyano, and the like.

The term "substituted" means that a moiety, *inter alia*, aromatic ring, alkyl chain, can have one or more of the hydrogen atoms replaced by a substituent. For example, 4-hydroxyphenyl is a "substituted aromatic carbocyclic ring", and 3-guanidinopropyl is a "substituted $C_3$ alkyl unit."

The term "hydrocarbyl" means "any unit which comprises carbon and hydrogen atoms, whether linear, branched, cyclic, and regardless of how many of the hydrogen atoms are substituted with a suitable "substituted" unit." Non-limiting examples of "hydrocarbyl" units include methyl, benzyl, 6-hydroxyoctanyl, m-chlorophenyl, 2-(N-methylamino)propyl, and the like. The following are non-limiting examples of moieties, which can replace hydrogen atoms on carbon to form a "substituted hydrocarbyl" unit:

i) $-NHCOR^{30}$;
ii) $-COR^{30}$;
iii) $-COOR^{30}$;
iv) $-COCH=CH_2$;
v) $-C(=NH)NH_2$;
vi) $-N(R^{30})_2$;
vii) $-NHC_6H_5$;
viii) $=CHC_6H_5$,
ix) $-CON(R^{30})_2$;
x) $-CONHNH_2$;
xi) -NHCN;
xii) -OCN;
xiii) -CN;
xiv) F, Cl, Br, I, and mixtures thereof;
xv) =O;
xvi) $-OR^{30}$;
xvii) -NHCHO;
xviii) -OH;
xix) $-NHN(R^{30})_2$;
xx) $=NR^{30}$;
xxi) $=NOR^{30}$;
xxii) $-NHOR^{30}$;
xxiii) -CNO;
xxiv) -NCS;
xxv) $=C(R^{30})_2$;
xxvi) $-SO_3M$;
xxvii) $-OSO_3M$;
xxviii) -SCN;
xxix) $-P(O)H_2$;
xxx) $-PO_2$;
xxxi) $-P(O)(OH)_2$;
xxxii) $-SO_2NH_2$;
xxxiii) $-SO_2R^{30}$;
xxxiv) $-NO_2$;
xxxv) $-CF_3$, $-CCl_3$, $-CBr_3$;
xxxvi) and mixtures thereof;

wherein $R^{30}$ is hydrogen, $C_1$-$C_{20}$ linear or branched alkyl, $C_6$-$C_{20}$ aryl, $C_7$-$C_{20}$ alkylenearyl, and mixtures thereof; M is hydrogen, or a salt forming cation. Suitable salt forming cations include, sodium, lithium, potassium, calcium, magnesium, ammonium, and the like. Non-limiting examples of an alkylenearyl unit include benzyl, 2-phenylethyl, 3-phenylpropyl, 2-phenylpropyl.

The term "inorganic carrier", means a carrier that comprises of non- or substantially non-carbon based backbones.

Odor Intensity Index Method: Odor Intensity Index is a value determined by expert graders who evaluate test chemicals for odor when such the pure chemicals are diluted at 1% in dipropylene glycol (DPG), odor-free solvent used in perfumery. This concentration percentage is representative of typical usage levels. Smelling strips, or so called "blotters", are dipped in test solutions and presented to expert panelists for evaluation. Expert panelists are assessors trained for at least six months in odor grading and whose gradings are checked for accuracy and reproducibility versus a reference on an on-going basis. For each amine compound, a panelist is presented two blotters: one reference (Me Anthranilate, unknown from the panelist) and the test sample. The panelist is asked to rank both smelling strips on the 0-5 odor intensity scale, 0 being no odor detected, 5 being very strong odor present.

The following represents Odor Intensity Index of some amine compounds suitable for use in the present invention. In each case, numbers are arithmetic averages among 5 expert panelists and the results are statistically significantly different at 95% confidence level:

| | |
|---|---|
| Methylanthranilate 1% (reference) | 3.4 |
| Ethyl-4-aminobenzoate (EAB) 1% | 0.9 |
| 1,4-bis-(3-aminopropyl)-piperazine (BNPP) 1% | 1.0 |

Protocol 1.1- Longevity Test: Each benefit delivery system that comprises a perfume raw material and an amine, is tested in accordance with the instant protocol. Each perfume aldehyde or ketone (P) found in such perfume raw material is tested with each amine to determine if the combination (PA) demonstrates a longevity that is greater than that obtained for P alone.

Multiple benefit agents may be tested together, at the same time, in the presence of multiple amines, as long as the analytical measurements are not compromised by such combination. By way of illustration, a benefit delivery system that contains six perfumes - three of which are aldehyde or a ketone perfumes ($P^1$, $P^2$ and $P^3$), and three of which are not aldehyde or ketone perfumes, and a single amine ($A^1$) requires the following single-variable test: ($P^1A^1$, $P^2A^1$ and $P^3A^1$) verses ($P^1$, $P^2$ and $P^3$), provided that said benefit agents are chromatographically separable such that the amount of each perfume aldehyde or ketone is easily determined in the presence of the other. Perfume aldehydes or ketones that are not chromatographically separable from one another must be run in separate tests. If, for example, $P^1$ and $P^3$ are not separable, then one of the following sets of tests is required:

I. ($P^1A^1$ and $P^2A^1$) vs. ($P^1$ and $P^2$), and ($P^3A^1$) vs. ($P^3$); or

II. ($P^2A^1$ and $P^3A^1$) vs. ($P^2$ and $P^3$), and ($P^1A^1$) vs. ($P^1$); or

III. ($P^1A^1$) vs. ($P^1$), and ($P^2A^1$) vs. ($P^2$), and ($P^3A^1$) vs. ($P^3$).

No P in any test should be present at a concentration greater than ten times the concentration of another P in the same test. In such a case, separate tests are indicated.

a.) Determination of the Concentration of Benefit Agent(s) and Amine in the Test Solution

The absolute concentration for the test solution (TS) to be used in a Benefit Agent Longevity Test (LT) is determined as follows.

The perfume aldehyde(s) or ketone(s) and amine(s) that are to be tested together are dissolved in 50:50 (v/v) ethanol:water at a concentration equal to that used in the benefit agent delivery system. The solution is closed to the atmosphere and aged for 24 hours at room temperature to obtain the initial test solution, designated $TS_0$. A 1.0 mL aliquot of $TS_0$ is pipetted onto a 4 cm diameter circle weighing 0.45 - 0.65 g (weight of circles in a given test should be the same $\pm$ 0.02 g) cut from an 86/14 cotton/poly terry wash cloth (obtained from EMC, 7616 Reinfold Drive, Cincinnati, OH 45237). The cloth, charged with test solution, is left open to the atmosphere under ambient conditions and subsequently analyzed via headspace gas chromatography (HSGC) to determine the amount of each perfume aldehyde or ketone in the headspace at each of the following times: 0.50, 1, 2, 4, 6, 8, and 24 hours.

The absolute concentration of perfume aldehyde(s) or ketone(s) and amine(s) to be used in the LT is the lowest concentration in a series of solutions based on $TS_0$ at which each perfume aldehyde or ketone in the TS is detected by

HSGC at no less than one of the designated time points. If this condition is not met by $TS_0$, the concentration of the test solution is doubled and the new solution ($TS_1$) is tested in the same mamler. The process is repeated until the condition is met. The concentration of perfume aldehyde(s) or ketone(s) and amine(s) in the test solution that meets the expressed condition ($TS_n$) is related to the concentration of the perfume aldehyde(s) or ketone(s) and amine(s) in $TS_0$ according to the following equation:

$$[P, A] \text{ in } TS_n = 2^{n} \cdot [P, A] \text{ in } TS_0;$$

where n = 0, 1, 2, 3...

b.) Headspace Gas Chromatography

Equipment required consists of:

1.) A trap containing a porous polymer having the ability to retain aroma materials, preferably Tenax TA 35/60 mesh.
2.) A source of pure helium.
3.) A headspace collector to contain the fabric circle and allow benefit agent to partition into the vapor headspace and reach equilibrium.
4.) GC-MS with headspace capabilities.

Suitable equipment is as referenced in S. Maeno, P.A. Rodriguez. J. Chromatography, A731 (1996) 201-215. It consists of equipment to transfer the equilibrated headspace vapors containing perfume aldehydes or ketones, which have been captured on a porous polymer, onto a GC for quantitative analysis. This equipment is able to heat the porous polymer trap containing the collected headspace, transferring the vapors to a cold trap cooled to < -100 °C (generally by liquid nitrogen). Following complete transfer to the cold trap, the cold trap is flash heated in a short period of time-typically about 1 minute-to a temperature of approximately 0 °C followed by a normal temperature gradient to about 280°C, resulting in the transfer of the headspace vapors directly onto a capillary GC column. A typical column is a 30 - 60 meter long with an i.d. of 0.18 - 0.32 mm, with a stationary phase composed of 100% dimethylpolysiloxane or (5%-phenyl)-methylpolysiloxane. The GC has the capability of quantitating and identifying said perfume aldehydes or ketones. Identification is accomplished via Mass Spectrometry and quantification is performed using a separate detector, such as an FID (flame ionization) or PID (photo ionization) detector.

c.) Longevity Test

A given test solution $TS_n$ meeting the condition described above is prepared. A second test solution $TS_c$ is prepared containing all the components of $TS_n$ at exactly the same concentrations as in $TS_n$ except that any amines have been removed. $TS_c$ serves as the control solution in the test.
Data is gathered for a given test solution (either $TS_c$ or $TS_n$) as follows: A 1.0 mL aliquot of TS is pipetted onto a 4 cm diameter circle cut from an 86/14 cotton/poly terry wash cloth. The cloth charged with TS is left open to the atmosphere under ambient conditions and subsequently analyzed via headspace gas chromatography (HSGC) to determine the amount of each benefit agent in the headspace at each of the following seven designated times: 0.50, 1, 2, 4, 6, 8, and 24 hours.
The conditions used for the analysis in each case are identical. The only difference for the two sets of data is that one set is obtained from a solution containing amine and the other set is obtained from a solution not containing amille.
A longevity benefit is confirmed for a particular P when the quantitative amount of the P in the headspace from $TS_n$ at any one of the seven designated times points is greater (statistically significant at 95% confidence) than the amount of the same P in the headspace from $TS_c$ at the corresponding time point.

d.) Example Results

The following table demonstrates the type of results that can be obtained from a Longevity Test. The data confirms a longevity benefit for $P^1$ (at t = 4 h, the area count from $TS_n > TS_c$) and $P^2$ (at t = 1 h, the area count from $TS_n > TS_c$) in the presence of $A^1$.

| | HSGC Area Count for Benefit Agent with and without $A^1$ | | | | | |
|---|---|---|---|---|---|---|
| | $P^1$ | | $P^2$ | | $P^3$ | |
| Time (h) | $TS_c$ | $TS_n$ | $TS_c$ | $TS_n$ | $TS_c$ | $TS_n$ |
| 0.50 | 12000 | 1000 | 8000 | 2000 | 30000 | 26000 |
| 1 | 6000 | 1500 | 400 | 1500 | 5000 | 4500 |
| 2 | 3000 | 2500 | 20 | 1000 | 850 | 700 |
| 4 | 1500 | 4000 | ND | 500 | 140 | 90 |
| 6 | 750 | 1500 | ND | 150 | 25 | ND |
| 8 | 220 | 500 | ND | 30 | ND | ND |
| 24 | ND | 50 | ND | ND | ND | ND |
| ND = Not detected. | | | | | | |

Amine

Applicants discovered that non-aromatic amines provide Applicants' delivery system with especially effective and efficient release characteristics. While not being bound by theory, Applicants believe that such characteristics are due to the substantial difference in the pKa of aromatic and non-aromatic amines. However, in general, suitable amines include mono-amines, such as a hydroxyamine or a polyamine so long as its molecular weight is greater than about 50 Daltons and so long as at least about 10% of its amino moieties are selected from the group consisting of primary amines, secondary amines and mixtures thereof. The amine comprises a primary amine moiety. The amine comprises, based on the total number of amine moieties in the amine, from about 15% to 100% primary amine moieties. Suitable hydroxyamines include hydroxyamines that have an average molecular weight of greater than about 100g/mole. Specific examples of such hydroxyamines include hydroxyamines selected from the group consisting of 2-hydroxyamines, 3-hydroxyamines and mixtures thereof. Suitable polyamines include polyamines that have an average molecular weight of from about 100 to about 2.10 x $10^6$ g/mol. In another aspect of Applicants' invention, suitable amines include amines having an Odor Intensity Index of less than that of a 1% solution of methylanthranilate in dipropylene glycol.

A general structure for suitable primary amines is as follows:

$$B\text{-}(NH_2)_n;$$

wherein B is a carrier material, and n is an index of value of at least 1. Compounds containing a secondary amine group have a structure similar to the above with the exception that the compound comprises one or more -NH- groups as well as $-NH_2$ groups. In one aspect of Applicants' invention, the amine compound, such as certain volatile amines, will not impart a sticky feel or undesired residue to a substrate that is treated with Applicants' invention.

The hydroxy amines of the present invention have the general formula:

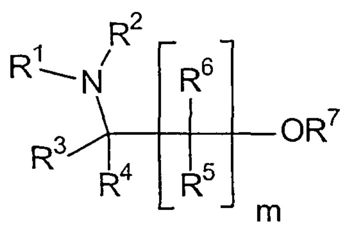

wherein, $R^1$ - $R^6$ units can be any substituted or unsubstituted hydrocarbyl unit, non-limiting examples include:

a) hydrogen;
b) $C_1$-$C_{10}$ substituted or unsubstituted linear alkyl;
c) $C_3$-$C_{10}$ substituted or unsubstituted branched alkyl;
d) $C_2$-$C_{10}$ substituted or unsubstituted linear alkenyl; as in the case of α, β, γ,

e) $C_3$-$C_{10}$ substituted or unsubstituted branched alkenyl;
f) $C_3$-$C_{15}$ substituted or unsubstituted cycloalkyl;
g) $C_4$-$C_{15}$ substituted or unsubstituted branched cycloalkyl;
h) $C_4$-$C_{15}$ substituted or unsubstituted cycloalkenyl;
i) $C_5$-$C_{15}$ substituted or unsubstituted branched cycloalkenyl;
j) $C_6$-$C_{15}$ substituted or unsubstituted aryl;
k) $C_6$-$C_{22}$ substituted or unsubstituted heterocyclicalkyl;
l) $C_6$-$C_{22}$ substituted or unsubstituted heterocyclicalkenyl;
m) and mixtures thereof;

alternatively the $R^3$ - $R^6$ units can be taken together to form a substituted or unsubstituted ring having in the ring from 3 to 10 carbon atoms; for example, $R^3$ and $R^5$ taken together can be fused ring comprising ketones. In one aspect of Applicants' invention, the index m is an integer from 1 to 3.

For the present invention $R^7$ is independently selected from any substituted or unsubstituted hydrocarbyl unit, non-limiting embodiments are selected from the group consisting of:

a) $R^6$;
b) hydroxyl;
c) a carbonyl comprising unit having the formula:

$$-(CH_2)_xCOR^8$$

wherein $R^8$ is:

i) -OH;
ii) $-OR^9$ wherein $R^9$ is hydrogen, $C_1$-$C_{15}$ substituted linear alkyl, $C_{11}$-$C_{15}$ unsubstituted linear alkyl, $C_1$-$C_{15}$ substituted branched alkyl, $C_{11}$-$C_{15}$ unsubstituted branched alkyl, $C_2$-$C_{22}$ substituted or unsubstituted linear alkenyl, $C_3$-$C_{22}$ substituted or unsubstituted branched alkenyl, or mixtures thereof, wherein said substitution is not halogen or thioalkyl; $R^9$ is methyl, $R^9$ is hydrogen and Z is oxygen or sulfur when an oxazolidine is formed from the methyl esters of serine, threonine, cysteine, and the like;
iii) $-N(R^{10})_2$ wherein $R^{10}$ is hydrogen, $C_1$-$C_6$ substituted or unsubstituted linear alkyl, $C_3$-$C_6$ substituted or unsubstituted branched alkyl, or mixtures thereof;
iv) $C_1$-$C_{22}$ substituted or unsubstituted linear alkyl;
v) $C_1$-$C_{22}$ substituted or unsubstituted branched alkyl;
vi) $C_2$-$C_{22}$ substituted or unsubstituted linear alkenyl;
vii) $C_3$-$C_{22}$ substituted or unsubstituted branched alkenyl;
viii) $C_3$-$C_{22}$ substituted or unsubstituted cycloalkyl;
ix) $C_6$-$C_{22}$ substituted or unsubstituted aryl;
x) $C_6$-$C_{22}$ substituted or unsubstituted heterocyclicalkyl;
xi) $C_6$-$C_{22}$ substituted or unsubstituted heterocyclicalkenyl; the index x is from 0 to 22;

d) alkyleneoxy units having the formula:

$$-[C(R^{11})_2]_y[C(R^{11})_2C(R^{11})_2O]_zR^{11}$$

wherein each $R^{11}$ is independently;

i) hydrogen;
ii) -OH;
iii) $C_1$-$C_4$ alkyl;
iv) or mixtures thereof;

two $R^{11}$ units can be taken together to form a $C_3$-$C_6$ spiroannulated ring, carbonyl unit, or mixtures thereof; y has the value from 0 to 10, z has the value from 1 to 50;
e) and mixtures thereof.

In one aspect of Applicants' invention, $R^2$ and $R^7$ are hydrogen. In another aspect of Applicants' invention, $R^1$ is selected from hydrogen and $C_1$-$C_{10}$ linear or branched alkyl. Non-limiting examples of such amines wherein m is 1

are represented by the following formula:

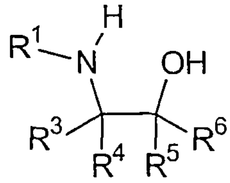

Non-linuting examples of suitable hydroxyamines include:

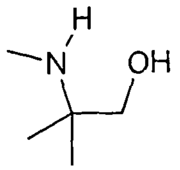
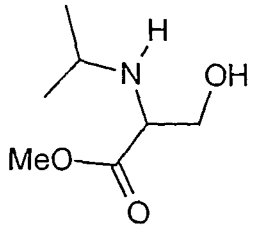
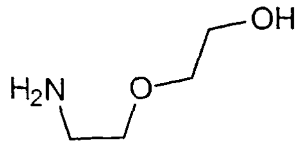
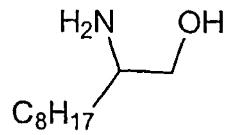

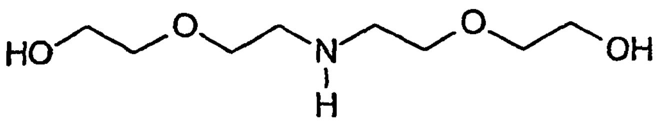
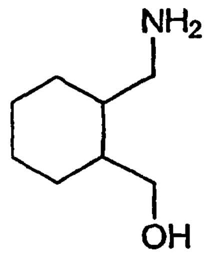

Suitable B carriers include both inorganic and organic carrier moieties.

Benefit Agent

Another essential component of the benefit agent delivery systems herein is a benefit agent. The benefit agents essentially used to form the delivery systems of this invention must be in the form of an aldehyde or ketone. It is understood that the genus of ketones includes those ketones, such as damascone, that comprise enone moieties. Such benefit agent can, for example, be selected from a flavor ketone or aldehyde, a pharmaceutical ketone or aldehyde, a biocontrol ketone or aldehyde, a perfume ketone or aldehyde and mixtures thereof. Perfume ketones and aldehydes are the most typical benefit agent used in Applicants' invention. A typical disclosure of suitable ketone and/or aldehydes, traditionally used in perfumery, can be found in "perfume and Flavor Chemicals", Vol. I and II, S. Arctander, Allured Publishing, 1994, ISBN 0-931710-35-5. This publication is also incorporated herein by reference.

The perfume ketones utilized in the benefit agent delivery systems herein can comprise any material which is chemically a ketone and which can impart a desirable odor or freshness benefit to surfaces. The perfume ketone component can, of course, comprise more than one ketone, i.e., mixtures of ketones. In one aspect of Applicants' invention, the perfume ketone is selected from the group consisting of buccoxime; iso jasmone; methyl beta naphthyl ketone; musk indanone; tonalid/musk plus; Alpha-Damascone, Beta-Damascone, Delta-Damascone, Iso-Damascone, Damascenone, Damarose, Methyl-Dihydrojasmonate, Menthone, Carvone, Camphor, Fenchone, Alpha-Ionone, Beta-Ionone, dihydro-Beta-Ionone, Gamma-Methyl so-called Ionone, Fleuramone, Dihydrojasmone, Cis-Jasmone, Iso-E-Super, Methyl- Cedrenyl-ketone or Methyl- Cedrylone, Acetophenone, Methyl-Acetophenone, Para-Methoxy-Acetophenone, Methyl-Beta-Naphtyl-Ketone, Benzyl-Acetone, Benzophenone, Para-Hydroxy-Phenyl-Butanone, Celery Ketone or Livescone, 6-Isopropyldecahydro-2-naphtone, Dimethyl-Octenone, Freskomenthe, 4-(1-Ethoxyvinyl)-3,3,5,5,-tetramethyl-Cyclohexanone, Methyl-Heptenone, 2-(2-(4-Methyl-3-cyclohexen-1-yl)propyl)-cyclopentanone, 1-(p-Menthen-6(2)-yl)-1-propanone, 4-(4-Hydroxy-3-methoxyphenyl)-2-butanone, 2-Acetyl-3,3-Dimethyl-Norbomane, 6,7-Dihydro-1,1,2,3,3-Pentamethyl-4(5H)-Indanone, 4-Damascol, Dulcinyl or Cassione, Gelsone, Hexalon, Isocyclemone E, Methyl Cyclocitrone, Methyl-Lavender-Ketone, Orivon, Para-tertiary-Butyl-Cyclohexanone, Verdone, Delphone, Muscone, Neobutenone, Plicatone, Veloutone, 2,4,4,7-Tetramethyl-oct-6-en-3-one, Tetrameran, hedione, and mixtures thereof. While in another aspect of Applicants' invention the perfume ketones is selected from the group consisting of Alpha Damascone, Delta Damascone, Iso Damascone, Carvone, Gamma-Methyl-Ionone, Iso-E-Super, 2,4,4,7-Tetramethyl-oct-6-en-3-one, Benzyl Acetone, Beta Damascone, Damascenone, methyl dihydrojasmonate, methyl cedrylone, hedione, and mixtures thereof.

Suitable perfume aldehydes can comprise any perfume material that is chemically an aldehyde, which can, like the perfume ketone component, impart a desirable odor or freshness benefit to surfaces. As with the perfume ketone benefit agents, the perfume aldehyde benefit agent component can comprise a single individual aldehyde or mixtures of two or more perfume aldehydes. Suitable perfume aldehyde materials for use in the delivery systems herein, whether by themselves or as part of a perfume aldehyde mixture, include melonal, triplal, Lugustral, adoxal; anisic aldehyde; cymal; ethyl vanillin; florhydral; helional; heliotropin; hydroxycitronellal; koavone; lauric aldehyde; lyral; methyl nonyl acetaldehyde; P. T. bucinal; phenyl acetaldehyde; undecylenic aldehyde; vanillin; 2,6,10-trimethyl-9-undecenal, 3-dodecen-1-al, alpha-n-amyl cinnamic aldehyde, 4-methoxybenzaldehyde, benzaldehyde, 3-(4-tert butylphenyl)-propanal, 2-methyl-3-(para-methoxyphenyl propanal, 2-methyl-4-(2,6,6-trimethyl-2(1)-cyclohexen-1-yl) butanal, 3-phenyl-2-propenal, cis-/trans-3,7-dimethyl-2,6-octadien-1-al, 3,7-dimethyl-6-octen-1-al, [(3,7-dimethyl-6-octenyl)oxy] acetaldehyde, 4-isopropylbenzyaldehyde, 1,2,3,4,5,6,7,8-octahydro-8,8-dimethyl-2-naphthaldehyde, 2,4-dimethyl-3-cyclohexen-1-carboxaldehyde, 2-methyl-3-(isopropylphenyl)propanal, 1-decanal; decyl aldehyde, 2,6-dimethyl-5-heptenal, 4-(tricyclo [5.2.1.0(2,6)]-decylidene-8)-butanal, octahydro-4,7-methano-1H-indenecarboxaldehyde, 3-ethoxy-4-hydroxy benzaldehyde, para-ethyl-alpha, alpha-dimethyl hydrocinnamaldehyde, alpha-methyl-3,4-(methylenedioxy)-hydrocinnamaldehyde, 3,4- methylenedioxybenzaldehyde, alpha-n-hexyl cinnamic aldehyde, m-cymene-7-carboxaldehyde, alpha-methyl phenyl acetaldehyde, 7-hydroxy-3,7-dimethyl octanal, Undecenal, 2,4,6-trimethyl-3-cyclohexene-1-carboxaldehyde, 4-(3)(4-methyl-3-pentenyl)-3-cyclohexen-carboxaldehyde, 1-dodecanal, 2,4-dimethyl cyclohexene-3-carboxaldehyde, 4-(4-hydroxy-4-methyl pentyl)-3-cylohexene-1-carboxaldehyde, 7-methoxy-3,7-dimethyloctan-1-al, 2-methyl undecanal, 2-methyl decanal, 1-nonanal, 1-octanal, 2,6,10-trimethyl-5,9-undecadienal, 2-methyl-3-(4-tertbutyl)propanal, dihydrocinnamic aldehyde, 1-methyl-4-(4-methyl-3-pentenyl)-3-cyclohexene-1-carboxaldehyde, 5 or 6 methoxy0hexahydro-4,7-methanoindan-1 or 2-carboxaldehyde, 3,7-dimethyloctan-1-al, 1-undecanal, 10-undecen-1-al, 4-hydroxy-3-methoxy benzaldehyde, 1-methyl-3-(4-methylpentyl)-3-cyclhexenecarboxaldehyde, 7-hydroxy-3,7-dimethyl-octanal, trans-4-decenal, 2,6-nonadienal, para-tolylacetaldehyde; 4-methylphenylacetaldehyde, 2-methyl-4-(2,6,6-trimethyl-1-cyclohexen-1-yl)-2-butenal, ortho-methoxycinnamic aldehyde, 3,5,6-trimethyl-3-cyclohexene carboxaldehyde, 3,7-dimethyl-2-methylene-6-octenal, phenoxyacetaldehyde, 5,9-dimethyl-4,8-decadienal, peony aldehyde (6,10- dimethyl-3-oxa-5,9-undecadien-1-al), hexahydro-4,7-methanoindan-1-carboxaldehyde, 2-methyl octanal, alpha-methyl-4-(1-methyl ethyl) benzene acetaldehyde, 6,6-dimethyl-2-norpinene-2-propionaldehyde, para methyl phenoxy acetaldehyde, 2-methyl-3-phenyl-2-propen-l-al, 3,5,5-trimethyl hexanal, Hexahydro-8,8-dimethyl-2-naphthaldehyde, 3-propyl-bicyclo[2.2.1]-hept-5-ene-2-carbaldehyde, 9-decenal, 3-methyl-5-phenyl-1-pentanal, methylnonyl acetaldehyde, 1-p-menthene-q-carboxaldehyde, citral, lilial and mixtures thereof. In one aspect of Applicants' invention perfume aldehydes are selected from the group consisting of citral, 1-decanal, benzaldehyde, florhydral, 2,4-dimethyl-3-cyclohexen-1-carboxaldehyde; cis/trans-3,7-dimethyl-2,6-octadien-1-al; heliotropin; 2,4,6-trimethyl-3-cyclohexene-1-carboxaldehyde; 2,6-nonadienal; alpha-n-amyl cinnamic aldehyde, alpha-n-hexyl cinnamic aldehyde, P.T. Bucinal, lyral, cymal, methyl nonyl acetaldehyde, trans-2-nonenal, lilial, trans-2-nonenal, and mixtures thereof.

Other suitable benefit agents include flavor ingredients including spices or flavor enhancers that contribute to the overall flavor perception of the product into which the benefit agent delivery system is incorporated. Pharmaceutical benefit agents including drugs. In one aspect of Applicants' invention a therapeutically acceptable amount of drug is empolyed. Biocontrol agents including biocides, antimicrobials, bactericides, fungicides, algaecides, mildewcides, disinfectants, sanitizer-like bleaches, antiseptics, insecticides, insect and/or moth repellant, vermicides, plant growth hormones, and the like. Antimicrobials including glutaraldehyde, cinnamaldehyde, and mixtures thereof. Typical insect and/or moth repellants such as citronellal, citral, N, N diethyl meta toluamide, Rotundial, 8-acetoxycarvotanacenone, and mixtures thereof. Other examples of insect and/or moth repellant for use as benefit agents herein are disclosed in US 4,449,987, 4,693,890, 4,696,676, 4,933,371, 5,030,660, 5,196,200, and "Semio Activity of Flavor and Fragrance molecules on various Insect Species", B.D. Mookherjee et al., published in <u>Bioactive Volatile Compounds from Plants</u>, ASC Symposium Series 525, R. Teranishi, R.G. Buttery, and H. Sugisawa, 1993, pp. 35-48. These publications are incorporated herein by reference.

<u>Delivery System Forms</u>

The benefit agent delivery systems herein may be based on the formation of a liquid or granular matrix. "Liquids" include fluids of density and viscosity that are conventional for liquids as well as gels and foams. Useful liquids may be

aqueous or non-aqueous. Water is typically the major component of the delivery systems that are in aqueous liquid form. Conventional non-aqueous solvents may be used to form the matrix for liquid delivery systems in non-aqueous form. Liquid products, i.e., those containing 10% or greater of water or other solvents, are highly preferred.

Delivery systems in granular form can be fashioned from any type of solid-state material that comprises particles or granules ranging in size from 1 μm to 100 mm. Thus the granular matrix can include particles ranging from very fine powder to agglomerates or tablets. The granular matrix furthermore can comprise either inert or active ingredients, or both, with respect to the function of the product into which the delivery system is to be incorporated.

Most typically, the liquid or granular matrix used to form the delivery systems herein will comprise the matrix for the liquid or granular end product into which the benefit agent delivery system will be incorporated and made a part of. Thus, for example, liquid or granular detergent compositions for laundry or hard surface cleaning will frequently comprise the liquid or granular matrix into which the amine-based compounds and benefit agents described herein will be separately added to form the delivery systems of tins invention.

Delivery System Preparation

It is an essential feature of the present invention that the amine compound and the benefit agent be added such that substantially no chemical reaction occurs between these materials prior to their contact with the liquid or granular matrix. For purposes of this invention, the amine-based compound and benefit agent are separately added to the system-forming matrix if the entire amounts of these components are combined with the matrix as discrete components. In particular, there must be essentially no chemical reaction between these two materials before they are combined with the matrix. Thus the amine compound and the benefit agent may be added to the matrix at separate times and/or from separate containers or from separate holding or delivery means. The amine compound and the benefit agent materials may even be mixed together prior to combination with the system-forming matrix so long as substantially no chemical reaction occurs between these materials prior to their contact with the system-forming matrix.

The benefit agent delivery system, especially in a granular form, can be prepared by simply admixing the amine-based compound and the benefit agent ketone and/or aldehyde under conditions which are sufficient to bring about combination, e.g., thorough admixture, of these components with the liquid or granular matrix. Frequently this admixing is carried out using high shear agitation. Temperatures of from about 40 °C to 65 °C may be utilized. Additional materials may also be added to the matrix in order to form the complete end product into which the delivery system is to be incorporated.

On a weight basis, the ratio of amine to benefit agent can vary widely, typically greater than about 1:5, more typically from about 1000:1 to about 1:1 for the two essential components. (amine compound and ketone/aldehyde benefit agent). In general, an excess of amine is desirable.

Adjunct Ingredients

Applicants' the various forms of Applicants' delivery system may contain adjunct ingredients including but not limited to water, surfactant, colorants and mixtures thereof.

Containers

Applicants' delivery may comprise one or more containers capable of containing the benefit agent and amine of the present invention in physical contact or sufficiently separate such that the benefit agent and amine are not in physical contact. Such one or more containers have separate compartments that may be empolyed to contain benefit agent and amine of the present invention in such a manner as to keep said benefit agent and amine sufficiently separate such that the benefit agent and amine are not in physical contact. One or more of said containers may comprise one or more spray dispensers capable of capable of dispensing said benefit agent and amine together or separately.

EXAMPLE

| | Weight % | | | |
|---|---|---|---|---|
| Ingredients | **1** | **2** | **3** | **4** |
| Pro-fragrance component | | | | |
| Pro-fragrance [1] | 1.0 | -- | -- | -- |
| Pro-fragrance [2] | 2.0 | -- | -- | -- |
| Pro-fragrance [3] | 2.0 | -- | -- | -- |

Table continued

| Free fragrance component | | | | |
|---|---|---|---|---|
| Daniascone | 0.0001 | 0.015 | -- | 0.01 |
| Melonal | 0.05 | 0.02 | -- | -- |
| Triplal | 0.01 | 0.03 | -- | -- |
| Beta-ionone | 0.01 | -- | -- | -- |
| Additional free fragrance raw materials containing at least one aldehyde / ketone[4] | 13.8 | 15.2 | 17.0 | 15.1 |
| Amine according to present invention | 0.09 | 0.5 | 1.5 | 0.03 |
| Carrier [5] | balance | balance | balance | balance |

1. Pro-fragrance which releases delta-damascone.
2. Pro-fragrance which releases melonal.
3. Pro-fragrance which releases triplal.
4. Conventional fragrance accord.
5. Ethanol:water mixture (between 100:0 and 50:50).

## Claims

**1.** A benefit agent delivery system suitable for delivering a benefit agent to a substrate, wherein the benefit agent delivery system comprises a benefit agent in the form of an aldehyde or ketone and an amine comprising a primary and/or secondary amine moiety, such that when said amine and said benefit agent are directly applied to a substrate, the benefit agent provides a benefit to the substrate for a longer period of time than when said amine is not present, wherein the amine comprises, based on the total number of amine moieties in the amine, from 15% to 100% primary amine moieties, and wherein the amine comprises a hydroxy moiety.

**2.** A benefit agent delivery system according any preceding claim wherein

a.) said benefit agent is selected from a perfume aldehyde, perfume ketone and mixtures thereof; and
b.) said benefit agent delivery system, when directly applied to a substrate, providing a benefit to the substrate for a longer period of time, as determined by Applicants' Test Protocol 1.1, than when said amine is not present.

**3.** The benefit delivery system of any preceding claim wherein said system comprises a sufficient amount of adjunct ingredients such that said benefit delivery system is a fine fragrance, home care perfume or freshening product, hard surface treating composition, beauty care product or hair acre product.

**4.** The benefit agent delivery system according to any preceding claim comprising one or more containers and wherein the benefit agent and amine are

a.) present in a single container in physical contact with each other; or separated from each other in a single container sufficiently such that said benefit agent and said amine are not in physical contact; or
b.) present in separate discrete containers.

**5.** The benefit agent delivery system according to Claim 4 wherein each container comprises at least one spray dispenser, said spray dispenser being capable of dispensing said benefit agent and amine:

a.) together; or
b.) separately.

**6.** A method of applying a benefit agent comprising a perfume aldehyde, perfume ketone and mixtures thereof to a substrate comprising contacting said substrate with said benefit agent before, during or after contacting said with an amine as defined in claim 1; said method preferably comprising directly contacting the substrate with the benefit agent delivery system of any preceding claim.

7. A benefit-containing substrate produced by the method of Claim 6.

## Patentansprüche

1. Wirkstoffabgabesystem, geeignet zum Abgeben eines Wirkstoffs an ein Substrat, wobei das Wirkstoffabgabesystem einen Wirkstoff in der Form eines Aldehyds oder Ketons und ein Amin umfasst, das eine primäre und/oder sekundäre Amineinheit umfasst, so dass, wenn das Amin und der Wirkstoff direkt auf ein Substrat aufgetragen werden, der Wirkstoff das Substrat für einen längeren Zeitraum mit einem Nutzen ausstattet, als wenn das Amin nicht vorliegt, wobei das Amin, basierend auf der Gesamtzahl von Amineinheiten in dem Amin, von 15 % bis 100 % primäre Amineinheiten umfasst und wobei das Amin eine Hydroxyeinheit umfasst.

2. Wirkstoffabgabesystem nach einem der vorstehenden Ansprüche, wobei

   a.) der Wirkstoff aus einem Duftstoffaldehyd, Duftstoffketon und Mischungen davon ausgewählt ist und
   b.) das Wirkstoffabgabesystem, wenn es direkt auf ein Substrat aufgetragen wird, das Substrat für einen längeren Zeitraum mit einem Nutzen ausstattet, wie durch das Prüfprotokoll 1.1 des Anmelders bestimmt, als wenn das Amin nicht vorliegt.

3. Wirkstoffabgabesystem nach einem der vorstehenden Ansprüche, wobei das System eine ausreichende Menge an Zusatzbestandteilen umfasst, so dass das Wirkstoffabgabesystem ein feiner Duftstoff, ein Geruchsstoff für die Hauspflege oder ein Erfrischerprodukt, eine Zusammensetzung zur Behandlung einer harten Oberfläche, ein Schönheitspflegeprodukt oder ein Haarpflegeprodukt ist.

4. Wirkstoffabgabesystem nach einem der vorstehenden Ansprüche, umfassend einen oder mehrere Behälter und wobei der Wirkstoff und das Amin

   a.) in einem einzigen Behälter in physikalischem Kontakt miteinander vorliegen oder in einem einzigen Behälter ausreichend getrennt voneinander sind, so dass der Wirkstoff und das Amin nicht in physikalischem Kontakt stehen; oder
   b.) in separaten, diskreten Behältern vorliegen.

5. Wirkstoffabgabesystem nach Anspruch 4, wobei jeder Behälter mindestens einen Sprühverteiler umfasst, wobei der Sprühverteiler dazu in der Lage ist, den Wirkstoff und das Amin folgendermaßen abzugeben:

   a.) zusammen oder
   b.) getrennt.

6. Verfahren zum Auftragen eines Wirkstoffs, umfassend ein Duftstoffaldehyd, Duftstoffketon und Mischungen davon, auf ein Substrat, umfassend das Inkontaktbringen des Substrats mit dem Wirkstoff vor, während oder nach dem Inkontaktbringen mit einem Amin wie in Anspruch 1 definiert; wobei das Verfahren vorzugsweise das direkte Inkontaktbringen des Substrats mit dem Wirkstoffabgabesystem nach einem der vorstehenden Ansprüche umfasst.

7. Nutzen aufweisendes Substrat, hergestellt durch das Verfahren nach Anspruch 6.

## Revendications

1. Système d'alimentation d'agent bénéfique approprié pour libérer un agent bénéfique sur un substrat, où le système d'alimentation d'agent bénéfique comprend un agent bénéfique sous la forme d'un aldéhyde ou d'une cétone et une amine comprenant un fragment amine primaire et/ou secondaire, de telle sorte que lorsque ladite amine et ledit agent bénéfique sont directement appliqués sur un substrat, l'agent bénéfique fournit un effet bénéfique au substrat pendant une plus longue période de temps que lorsque ladite amine n'est pas présente, où l'amine comprend, basé sur le nombre total de fragments amine dans l'amine, de 15 % à 100 % des fragments amine primaire, et où l'amine comprend un fragment hydroxy.

2. Système d'alimentation d'agent bénéfique selon l'une quelconque des revendications précédentes, où

a.) ledit agent bénéfique est choisi parmi un aldéhyde de parfum, une cétone de parfum et leurs mélanges; et
b.) ledit système d'alimentation d'agent bénéfique, lorsqu'il est directement appliqué sur un substrat, fournissant un effet bénéfique au substrat pendant une plus longue période de temps, comme déterminé par le Protocole de Test 1.1 des Demandeurs, que lorsque ladite amine n'est pas présente.

**3.** Système d'alimentation d'agent bénéfique selon l'une quelconque des revendications précédentes, où ledit système comprend une quantité suffisante d'ingrédients additifs de telle sorte que ledit système d'alimentation d'effet bénéfique est un parfum fin, un parfum pour le soin de la maison ou un produit rafraîchissant, une composition pour le traitement de surface dure, un produit de soin de beauté ou un produit de soin des cheveux.

**4.** Système d'alimentation d'agent bénéfique selon l'une quelconque des revendications précédentes, comprenant un ou plusieurs récipients et où l'agent bénéfique et l'amine sont

a.) présents dans un seul récipient en contact physique l'un avec l'autre; ou indépendants l'un de l'autre dans un seul récipient suffisamment de telle sorte que ledit agent bénéfique et ladite amine ne soient pas en contact physique; ou
b.) présents dans des récipients individuels indépendants.

**5.** Système d'alimentation d'agent bénéfique selon la revendication 4, dans lequel chaque récipient comprend au moins un atomiseur, ledit atomiseur, étant susceptible de distribuer lesdits agent bénéfique et amine:

a.) conjointement; ou
b.) séparément.

**6.** Procédé d'application d'un agent bénéfique comprenant un aldéhyde de parfum, une cétone de parfum et leurs mélanges sur un substrat comprenant la mise en contact dudit substrat avec ledit agent bénéfique avant, durant ou après mise en contact dudit avec une amine comme défini dans la revendication 1; ledit procédé comprenant de préférence la mise en contact directe du substrat avec le système d'alimentation d'agent bénéfique selon l'une quelconque des revendications précédentes.

**7.** Substrat contenant un effet bénéfique produit par le procédé selon la revendication 6.